# EUROPÄISCHE PATENTSCHRIFT

(11) **EP 0 416 426 B1**
(45) Veröffentlichungstag und Bekanntmachung des Hinweises auf die Patenterteilung: **15.11.1995**
(21) Anmeldenummer: 90116420.2
(22) Anmeldetag: 28.08.1990
(51) Int. Cl.: C12Q 1/68

(54) **Nachweis von Influenza-A-Virus durch Polymerase-Kettenreaktion (PCR) nach reverser Transkription eines Bereichs des viralen Hämagglutinin-Gens**
Detection of influenza-A-virus by polymerase chain reaction following the reverse transcription of a domain of the viral hemagglutinine gene
Détection du virus A de la grippe par la réaction en chaîne de la polymérase suivant la transcription inverse d'un domaine du gène viral de l'hémagglutinine

(30) Priorität: 02.09.1989 DE 3929144
(43) Veröffentlichungstag der Anmeldung: 13.03.1991
(73) Patentinhaber: BEHRINGWERKE Aktiengesellschaft, 35001 Marburg (DE)
(72) Erfinder: Cerutti, Peter A.,Dr.Prof., CH-1012 Pully/Lausanne (CH); Bressoud, Albéric, CH-1006 Lausanne (CH)

(56) Entgegenhaltungen:
- Proceedings of the Nat. ACADEMY OF SCIENCES USA, Band 87, Juni 1990; A.RAJAKUMAR et al., Seiten 4154-4158 #
- Science,Band 239, 29 Januar 1988; R.K. SAIKI et al., Seiten 487-491 #
- Trends in Genetics,Band 5, Nr. 6, Juni 1989, Elsevier Science Publishers Ltd.,New York, NY (US); T.J. WHITE et al., Seiten 185-189 #
- Journal of the American Vet. Med. Association,Band 194, Nr. 12, 1989, 126thAnnual American Vet. Med. Association Meeting, Orlando, FL (US), 15-19 Juli1989, Chicago, IL (US); M.L. PERDUE, Seite 1795; and Y. KAWAOKA, Seite 1795 #
- Biochemical & Physical Research Communications. Vol. 167, 1990. pp. 425-430. Bressoud et al.
- Nature, vol. 292. 1981. pp.72-75.Winter et al.
- Nucleic acids Research, vol. 11pp. 7191-7202, 1983. Raymond et al.
- Journal General Virology, vol 70, 1989.pp. 299-313. Cox et al.

## Beschreibung

Die Empfindlichkeit des Nachweises von Influenza-A-Virus konnte wesentlich dadurch gesteigert werden, daß unter Einsatz von Oligonukleotidsequenzen (Amplimeren) aus der einigermaßen konservierten Region des viralen Hämagglutinin-Gens nach vorangegangener reverser Transkription (RT) eine Polymerase-Kettenreaktion (PCR) durchgeführt wurde. Die reverse Transkription und die PCR sind dabei unempfindlich gegen einzelne Mutationen durch Basenaustausch, sofern nicht das 3′-Ende der Amplimere betroffen ist.

Das Influenza-A-Virus kommt bei Menschen und als artspezifischer Erreger auch bei Tieren vor und ist die Ursache der in größeren Abständen auftretenden Pandemien. Die Pandemien werden durch den Antigenwechsel des Influenza-A-Virus ausgelöst. Es entstehen neue Antigenvarianten, gegen welche die Bevölkerung keine Immunität aufweist, und die sich aus diesem Grunde äußerst rasch ausbreiten und mit einer hohen Mortalität verbunden sind.

Deshalb ist der schnelle und zuverlässige Nachweis einer Influenza-A-Virus Infektion sehr wichtig. Im allgemeinen wird versucht, die Labordiagnose über den Virus-Nachweis zu führen. Die Anzüchtung des Influenza-Virus gelingt allerdings nur in den ersten Krankheitstagen.

Nach Verimpfung von Nasen- oder Rachensekret auf Affennierenzellkulturen macht sich die Virusvermehrung durch die Hämadsorption von Meerschweinchenerythrozyten bemerkbar. Die Spezifität kann durch Hemmung der Hämadsorption mit Hilfe eines spezifischen Immunserums geprüft werden. Ein zytopathischer Effekt tritt nicht immer auf. Es gibt Influenzavirusstämme, die leichter durch Verimpfung in die Amnionhöhle des embryonierten Hühnereies isoliert werden können.

Es sind Versuche durchgeführt worden, um auch bei der Influenza eine Schnelldiagnose zu erreichen. Zu diesem Zweck wird das Nasen-Rachensekret auf Objektträger ausgestrichen und das Virusantigen mit der direkten oder indirekten Immmunofluoreszenz unter Verwendung spezifischer Immunseren nachgewiesen. Diese Untersuchung benötigt nur wenige Stunden, ist aber oft nicht empfindlich genug, besonders wenn die Probe spät genommen wurde.

Es stellte sich deshalb die Aufgabe, die Empfindlichkeit der bisherigen Virus-Nachweismethoden für Influenza-A-Virus zu verbessern. Ein bekanntes Nachweisverfahren war beispielsweise die Polymerase-Ketten-reaktion (1, 2). H1 war darüberhinaus als wichtiges Antigen des Influenza-A-Virus bekannt und seine Sequenz beschrieben (3, 4). Wir haben gefunden, daß durch reverse Transkription und nachfolgende Amplifikation durch die Polymerase-Kettenreaktion mittels geeigneter Oligonukleotide aus einem weitgehend konservierten Bereich des viralen Hämagglutinin-Gens auf dem vierten Segment des Virusgenoms sich die Empfindlichkeit des Antigen-Nachweises wesentlich steigern läßt. Basenaustausche innerhalb des Bereichs, in dem vorgenannten Oligonukleotide (Amplimere) liegenden Bereichs stören dabei nicht solange nicht die 3′-Enden der Amplimere betroffen sind. Am günstigsten zur reversen Transkription und Amplifikation hat sich der Bereich von Position 96 bis Position 536 des Hämagglutinins H1 erwiesen, jedoch sind auch die Bereiche ± 80 Nukleotide von den angegebenen Positionen geeignet. Die Auswahl des Hämagglutinin-H1-Gens und insbesondere dieser Region hat den Vorteil, daß - von Ausnahmen wie oben skizziert abgesehen - alle Serotypen des Influenza-A-Virus erfaßt werden.

Die Erfindung betrifft folglich eine Nachweismethode für Influenza-A-Virus durch reverse Transkription eines Bereichs des Hämagglutinin-Gens H1, anschließende Amplifikation durch PCR unter weiterer Verwendung der zur RT eingesetzten Amplimere und anschließenden Nachweis der amplifizierten cDNA, vorzugsweise als Direktnachweis durch Anfärben nach gelelektrophoretischer Auftrennung. Jedoch kann die amplifizierte cDNA auch durch Radioisotope oder in einem ELISA nachgewiesen werden, falls vorher radiomarkierte bzw. biotinylierte Desoxyribonukleotidtriphosphate eingesetzt wurden. Die Amplimere wurden als Oligonukleotide mit einer Länge von etwa 20 Nukleotiden im Bereich der Position 96 ± 80 bis 536 ± 80, bevorzugt der Position 96 ± 20 bis 536 ± 20 und besonders bevorzugt der Position 96 bis 536 des Hämagglutinin-Gens H1 ausgewählt. Die PCR wird vorzugsweise bei 59 °C und 91°C und Zykluszeiten von je etwa einer Minute besonders bevorzugt 97 sec. bei 59 °C und 85 sec. bei 91 °C in Gegenwart von 1 mM Desoxyribonukleotidtriphosphaten, 3 % Dimethylsulfoxid und 0.8 µg jedes Amplimers in einem Gesamtvolumen von 25 µl durchgeführt.

Die Erfindung ist schließlich in den Beispielen und den Patentansprüchen beschrieben.

### Beispiele:

### 1. Isolierung viraler RNA

Influenza-Virus H1 (A/WSN/33) Suspensionen in PBS mit 0.2% Albumin (1) bzw. 28 nasopharyngeale Lavagen von mutmaßlich Infizierten (2) oder-Virus aus infizierten Zellen nach Beschallung (3) wurden dreimal mit saurer Guanidin-Thiocyanat-Phenol-Chloroform-Lösung (P. Chomczynski and N. Sacchi, (1987), Analytical Biochemistry 162, 156-159) extrahiert. Virale RNA wurde in Gegenwart eines t-RNA "Carriers" (1 µg/ml) durch Zugabe von 2.5 Volumina 90%igem gepuffertem Äthanol bei -20°C gefällt. Nach Zentrifugation wurde der Niederschlag mit 80%igem gepuffertem Äthanol gewaschen, getrocknet und in sterilem Wasser resuspendiert. Die Ultraschallbehandlung bei (3) erfolgte unter Paraffin-Öl 15 sec. bei Stufe 2 eines Branson Ultraschallgeräts B15.

### 2. Reverse Transkription

Die reverse Transkription wurde bei 42°C für 30 Minuten in einem Gesamtvolumen von 5 µl vorgenommen. Darin waren in einem Puffer von 50 mM Tris in HCl pH 8.3 50 mM KCl, 7 mM MgCl₂, 170 µg/ml BSA, 1 mM je dATP, dTTP, dCTP, dGTP, 0.1% Triton® X-100, 1 mM DTT und 10 mM 2-Mercaptoäthanol (Puffer A) je 0.4 µg der beidem Amplimere, 7 U AMV Reverse Transkriptase (Pharmacia) und 10 U RNAsin (Promega) enthalten.

### 3. Amplifikation durch PCR

Die vorstehend gewonnene cDNA wurde wie folgt amplifiziert:In 25 µl Gesamtvolumen in Puffer A ohne Triton® -100 und DTT aber mit 3 % DMSo und 0.8 µg jedes Amplimers wurde 1 U Taq-Polymerase gegeben (Biofinex, Praroman, Fribourg (Schweiz)).

Amplifiziert wurde durch 30 bis 40 Zyklen mit einem Ampligene-Apparat (Moretronic, Schweiz) bei 59°C (je 97 sec.) und 91°C (je 85 sec.).

### 4. Nachweis der amplifizierten cDNA

Amplifizierte Proben wurden in einem 2%igen Agarose-Gel in 10 mM Tris-HCl pH 7.4 0.1 mM EDTA und 0.7 µg/ml Ethidium-Bromid unter UV-Licht nachgewiesen.

Die Tabelle zeigt (unterstrichen) die Position der eingesetzten Amplimere, die auf einem DNA-Synthesizer (Bio Applied Systems) synthetisiert wurden und durch Sephadex®NAP-24 Säulen gereinigt wurden.

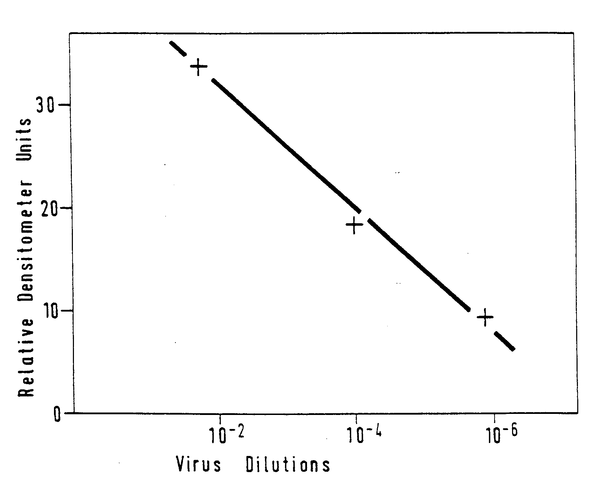

Die Figur zeigt die Ergebnisse eines Modellversuches, bei dem Verdünnungen (10⁻² bis 10⁻⁶, Abszisse) einer Virussuspension eingesetzt wurde und die Intensität der 441 Basenpaare (bp)-langen Bande des Agarosegels (Ordinate) aufgetragen sind.

Bei 28 frischen nasopharyngealen Lavagen möglicherweise infizierter Personen wurde zweimal die 441 bp Bande nachgewiesen. Zur internen Kontrolle wurde jeweils ein 201 bp langes Fragment des zellulären β-Globin Gens (Position 717 bis 926, siehe J.C. Schimenti und C.H. Duncan, Nucl. Acids. Res. 12, (1984), 1641-1653) koamplifiziert.

TAB.

44 CCUACUGGUCCUGUU AUGUGCACUUGCAGC UGCAGAUGCAGACAC
89 AAUAUGUAUAGGCUA CCAUGCGAACAAUUC AACCGACACUGUUGA
134 CACAGUACUCGAGAA GAAUGUGACAGUGAC ACACUCUGUUAACCU
179 GCUCGAAGACAGCCA CAACGGAAAACUAUG UAGAUUAAAAGGAAU
224 AGCCCCACUACAAUU GGGGAAAUGUAACAU CGCCGGAUGGCUCUU
269 GGGAAACCCAGAAUG CGACCCACUGCUUCC AGUGAGAUCAUGGUC
314 CUACAUUGUAGAAAC ACCAAACUCUGAGAA UGGAAUAUGUUAUCC
359 AGGAGAUUUCAUCGA CUAUGAGGAGCUGAG GGAGCAAUUGAGCUC
404 AGUGUCAUCAUUCGA AAGAUUCGAAAUAUU UCCCAAAGAAAGCUC
449 AUGGCCCAACCACAA CACAAACGGAGUAAC GGCAGCAUGCUCCCA
494 UGAGGGGAAAAGCAG UUUUUACAGAAAUUU GCUAUGGCUGACGGA
539 GAAGGAGGGCUCAUA CCCAAAGCUGAAAAA UUCUUAUGUGAACAA

### Literaturstellen:

1: White, T. J. et al (1989) Trends in Genetics, Vol. 5, No. 6, pp. 185-189
2: Saiki, R. K. et al. (1988) Science, Vol. 239, pp. 487-491
3: Raymond, F. L. (1983) Nucleic Acid Res., Vol. 11, No. 20, pp. 7191-7202
4: Winter, G. (1981) Nature, Vol. 292, pp. 72-75

## Patentansprüche (Patentansprüche für folgende(n) Vertragsstaat(en): AT, BE, CH, DE, DK, FR, GB, GR, IT, LI, LU, NL, SE)

1. Verfahren zum Nachweis von Influenza-A-Virus, dadurch gekennzeichnet, daß zur reversen Transkription (RT) und anschließender Polymerase-Kettenreaktion (PCR) als Amplimere Oligonukleotide des Hämagglutinin-Gens H1 von etwa 20 Basenpaaren (bp) Länge der Position zwischen 96 ± 80 und 536 ± 80 eingesetzt werden.

2. Verfahren nach Anspruch 1, dadurch gekennzeichnet, daß als Amplimere Oligonukleotide von etwa 20 bp der Position zwischen 96 ± 20 und 536 ± 20 eingesetzt werden.

3. Verfahren nach Anspruch 1, dadurch gekennzeichnet, daß die Amplimere AUAGGCUACCAUGCGAACAA und GAAAUUUGCUAUGGCUGACG eingesetzt werden.

4. Reagenzkombination zum Nachweis von Influenza-A-Virus, dadurch gekennzeichnet, daß sie Amplimere enthält, welche etwa 20 bp der Position zwischen 96 ± 80 und 536 ± 80 des Hämagglutinin-Gens H1 überspannen.

5. Reagenzkombination nach Anspruch 4, dadurch gekennzeichnet, daß die Amplimere AUAGGCUACCAUGCGAACAA und GAAAUUUGCUAUGGCUGACG eingesetzt werden.

## Patentansprüche (Patentansprüche für folgende(n) Vertragsstaat(en): ES)

1. Verfahren zum Nachweis von Influenza-A-Virus, dadurch gekennzeichnet, daß zur reversen Transkription (RT) und anschließender Polymerase-Kettenreaktion (PCR) als Amplimere Oligonukleotide des Hämagglutinin-Gens H1 von etwa 20 Basenpaaren (bp) Länge der Position 96 ± 80 und 536 ± 80 eingesetzt werden.

2. Verfahren nach Anspruch 1, dadurch gekennzeichnet, daß als Amplimere Oligonukleotide von etwa 20 bp der Position 96 ± 20 und 536 ± 20 eingesetzt werden.

3. Verfahren nach Anspruch 1, dadurch gekennzeichnet, daß die Amplimere AUAGGCUACCAUGCGAACAA und GAAAUUUGCUAUGGCUGACG eingesetzt werden.

## Claims (Claims for the following Contracting State(s): AT, BE, CH, DE, DK, FR, GB, GR, IT, LI, LU, NL, SE)

1. A method for the detection of influenza A virus, which comprises the use of oligonucleotides of the hemagglutinin gene H1 with a length of about 20 base pairs (bp) from a position between 96 ± 80 and 536 ± 80 as amplimers for reverse transcription (RT) and subsequent polymerase chain reaction (PCR).

2. The method as claimed in claim 1, wherein oligonucleotides of about 20 bp from a position between 96 ± 20 and 536 ± 20 are used as amplimers.

3. The method as claimed in claim 1, wherein the amplimers **AUAGGCUACCAUGCGAACAA** and **GAAAUUUGCUAUGGCUGACG** are used.

4. A reagent combination for the detection of influenza A virus which contains amplimers which cover about 20 bp from a position between 96 ± 80 and 536 ± 80 of the hemagglutinin gene H1.

5. A reagent combination as claimed in claim 4, wherein the amplimers **AUAGGCUACCAUGCGAACAA** and **GAAAUUUGCUAUGGCUGACG** are used.

## Claims (Claims for the following Contracting State(s): ES)

1. A method for the detection of influenza A virus, which comprises the use of oligonucleotides of the hemagglutinin gene H1 with a length of about 20 base pairs (bp) from a position between 96 ± 80 and 536 ± 80 as amplimers for reverse transcription (RT) and subsequent polymerase chain reaction (PCR).

2. The method as claimed in claim 1, wherein oligonucleotides of about 20 bp from a position between 96 ± 20 and 536 ± 20 are used as amplimers.

3. The method as claimed in claim 1, wherein the amplimers **AUAGGCUACCAUGCGAACAA** and **GAAAUUUGCUAUGGCUGACG** are used.

## Revendications (Revendications pour l'(les) Etat(s) contractant(s) suivant(s): AT, BE, CH, DE, DK, FR, GB, GR, IT, LI, LU, NL, SE)

1. Procédé pour la détection du virus A de la grippe, caractérisé en ce que, pour la transcription inverse (RT) et la réaction de polymérisation en chaîne (PCR) subséquente, on utilise comme amplimères des oligonucléotides du gène H1 de l'hémagglutinine d'une longueur d'environ 20 paires de bases (pb) localisés entre les positions 96 ± 80 et 536 ± 80.

2. Procédé selon la revendication 1, caractérisé en ce que, comme amplimères, on utilise des oligonucléotides d'environ 20 pb localisés entre les positions 96 ± 20 et 536 ± 20.

3. Procédé selon la revendication 1, caractérisé en ce que l'on utilise les amplimères AUAGGCUACCAUGCGAACAA et GAAAUUUGCUAUGGCUGACG.

4. Association de réactifs pour la détection du virus A de la grippe, caractérisée en ce qu'elle contient des amplimères qui recouvrent environ 20 pb localisées entre les positions 96 ± 80 et 536 ± 80 du gène H1 de l'hémagglutinine.

5. Association de réactifs selon la revendication 4, caractérisée en ce que l'on utilise les amplimères AUAGGCUACCAUGCGAACAA et GAAAUUUGCUAUGGCUGACG.

## Revendications (Revendications pour l'(les) Etat(s) contractant(s) suivant(s): ES)

1. Procédé pour la détection du virus A de la grippe, caractérisé en ce que, pour la transcription inverse (RT) et la réaction de polymérisation en chaîne (PCR) subséquente, on utilise comme amplimères des oligonucléotides du gène H1 de l'hémagglutinine d'une longueur d'environ 20 paires de bases (pb) localisés entre les positions 96 ± 80 et 536 ± 80.

2. Procédé selon la revendication 1, caractérisé en ce que, comme amplimères, on utilise des oligonucléotides d'environ 20 pb localisés entre les positions 96 ± 20 et 536 ± 20.

3. Procédé selon la revendication 1, caractérisé en ce que l'on utilise les amplimères AUAGGCUACCAUGCGAACAA et GAAAUUUGCUAUGGCUGACG.
